# EUROPEAN PATENT APPLICATION

(11) **EP 1 721 629 A1**
(43) Date of publication of application: **15.11.2006**
(21) Application number: 05003518.7
(22) Date of filing: 18.02.2005
(51) Int. Cl.: A61M 16/00

(54) **Method and apparatus for intermittent hypoxic training**

(71) Applicant: Bassovitch, Oleg, Highett, Victoria 3190 (AU)
(72) Inventor: Bassovitch, Oleg, Highett, Victoria 3190 (AU)
(74) Representative: Lipscombe, Martin John

(57) **Abstract**

An apparatus for the delivery of hypoxic air to a user comprising a biofeedback means where at least one physiologically measurable parameter of the user is substantially constantly measured by a monitoring means and the measured data transmitted to a control means, where the control means comprises:
i) means for comparing the measured data of the at least one physiological parameter with a pre-set target value for the parameter; and
ii) adjustment means to vary the oxygen concentration in the hypoxic air delivered to the user in response to the transmitted data.

## Description

### Field of the Invention

This invention relates to improved method and apparatus for the treatment of mammals using pre-acclimatisation to a simulated altitude environment by the process of hypoxic air breathing. More specifically, the present invention relates to improved apparatus for the administration of Intermittent Hypoxic Training (IHT), which involves subjecting a mammalian user to repeated exposures of normobaric hypoxic air breathing, alternated with ambient air breathing and to a method employing the improved apparatus.

### Background to the Invention

Pre-acclimatisation to simulated altitude conditions (reduced oxygen breathing) is known to produce a cluster of beneficial alterations to mammalian physiology. Short-term respiration with reduced oxygen air initiates a number of compensatory mechanisms in the mammalian body. A course of repeated short-term hypoxia exposures has been shown to stimulate erythropoietin and haemoglobin production, stimulate respiratory muscles and ventilation, produce hypotensive and vasodilative effects, reduce free-radical formation in the body and also increase the body's antioxidant enzymatic capacity.

These physiological responses are utilised very effectively in the training and treatment of athletic users, for improved general health and wellbeing and for the treatment of various degenerative disorders found in mammals in general.

The most commonly used treatment protocol for Intermittent Hypoxic Training (IHT) comprises subjecting the user to repeated exposures of hypoxic air breathing, alternated with breathing ambient or normoxic air. During a normal training course, the oxygen concentration in the hypoxic air supplied to the user is decreased gradually from 15 - 12 % of oxygen by volume down to 8 - 12 % of oxygen by volume in order to provide the user with a step-wise reduction so as to avoid any undesired symptoms that may arise as a result of over-training.

In a normal treatment program, IHT is delivered to the user 2 - 7 times per week for a period of 2 - 4 weeks and the duration of one session is usually in the range of between 40 - 120 minutes.

Typically, the user's physiological response is monitored over the course of treatment by a pulse oximeter, which is a device that measures arterial oxygen saturation (SpO₂) and heart rate (HR). Optionally, other physiological parameters may also be monitored, for instance blood pressure, heart activity by way of an electrocardiogram, etc. Careful monitoring of a user's physiological parameters allows the undesirable effects of over-dosing to be avoided.

A number of systems and devices for delivery of Intermittent Hypoxic Training (IHT) have been proposed in the past including disclosures in Soviet Union Patents, SU1264949, SU1313444, SU1335294, SU950406 and SU1801440; Russian Patents RU2158610, RU2004261, RU2019199, RU2201769 and RU2115366 and United States Patent No.'s 05101819, 05850833 and 6,009,870.

All of the above prior devices focus on oxygen concentrations in hypoxic air as delivered to respiratory system of the user. A problem associated with each of these earlier devices is that they follow the standard pre-set approach and do not take into consideration each individual user's response thereby resulting in an inability to tailor or maximise delivery of and hence the benefits of IHT to each individual user's needs.

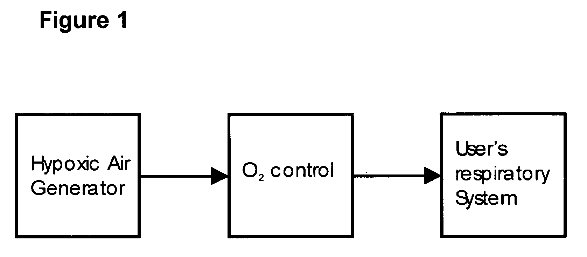

The earlier IHT devices described in the above mentioned patents can be schematically summarised as shown in Figure 1. In such devices the "Hypoxic Air Generator" produces hypoxic air, the oxygen composition in the hypoxic air is selected and set and controlled by "O₂ control" and delivered to the "User" or the "User's Respiratory System".

It has been widely accepted by workers in this field, for example, as reported by Levine BD and Stray-Gundersen J, "Living high-training low: Effect of moderate-altitude acclimatization with low-altitude training on performance," J. Appl. Physiol. (1997) Jul; 83(1):102-123; Stray-Gundersen J and Levine BD "Living high and training low can improve sea level performance in endurance athletes,". Br. J. Sports Med., (1999) Jun; 33(3): 150-1; Levine B.D. in "Intermittent hypoxic training: fact and fancy," High Alt. Med. Biol. (2002) Summer; 3(2): 177-93; and Serebrovskaya T.V., "Intermittent hypoxia research in the former Soviet Union and the Commonwealth of independent States: History and review of the concept and selected applications," High Alt. Med. Biol., (2002) Summer; 3(2): 205-21, that the optimal regime for a particular individual can readily be determined using parameters such as the timing, duration and dosage of IHT exposure. While timing and duration of the session at pre-set O₂ levels was easy to determine and control, the selection of an optimal dosage has hitherto remained the most difficult task to achieve.

It has now been discovered that different users exposed to the same oxygen level in a pre-determined inspired hypoxic gas mixture (FiO₂) respond very differently. Further, it has surprisingly been found that the same user responds demonstrably differently on different days to the same concentration of oxygen in the delivered hypoxic air (FiO₂) as measured in the SpO₂ and/or HR parameters of the user, respectively.

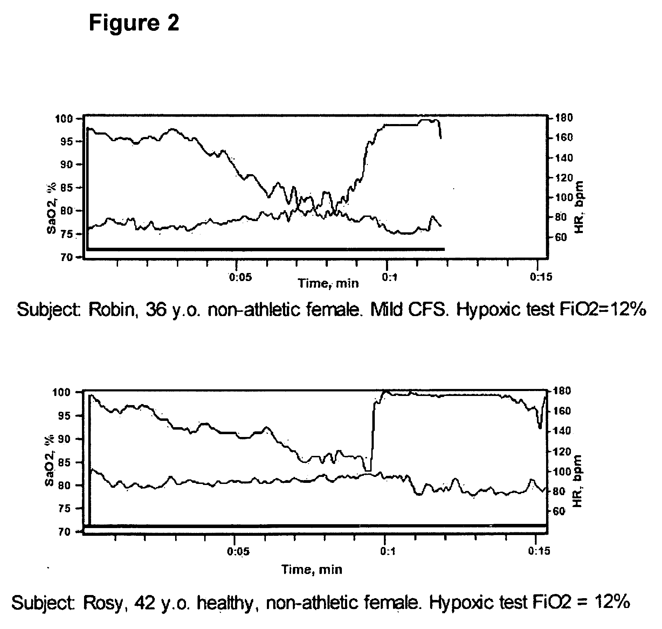

Using a typical IHT device as shown in Figure 1, Figure 2 illustrates the results of hypoxic tests conducted on two non-athletic users exposed to the same level of hypoxic hypoxia for 9 to 10 minutes. Both subjects received hypoxic air composed of 12% of oxygen by volume with the balance being made up of nitrogen (FiO₂ = 0.12) at sea level. In subject "Robin", the SpO₂ decreased down to 78 % within 8 minutes, whereas subject "Rosy" was not able to desaturate below 85 % under the same time and level of exposure.

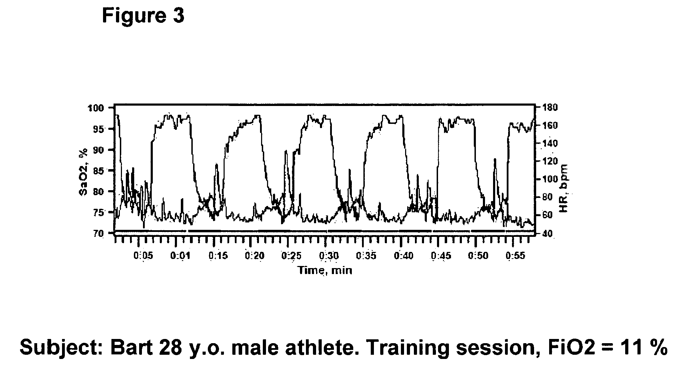

In a further example, a typical training session for athletic subject "Bart" is shown in Figure 3. During a 5-minute hypoxic air exposure to FiO₂ = 0.11, alternated with ambient air breathing, his arterial oxygen saturation repeatedly dropped below 73%.

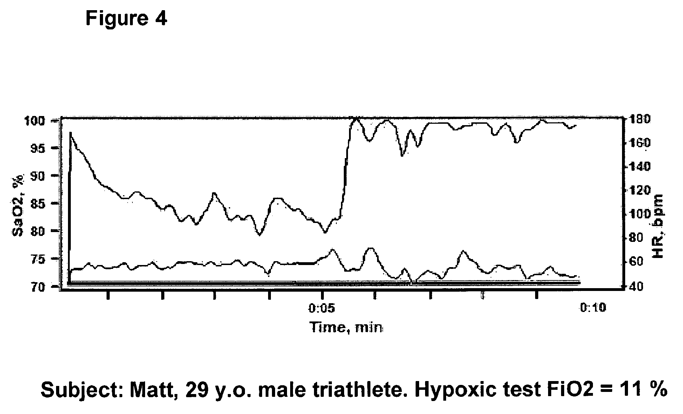

The same composition of hypoxic air (FiO₂ = 0.11) was used by an athletic subject "Matt" and caused a maximum desaturation of only 80% over a time period, as shown in Figure 4.

It can therefore be seen from the devices and methods of the prior art that the supply of a known composition of hypoxic air to the user does not ensure a predictable result or benefit for the user. As each course of treatment is individually prescribed, depending upon each user's requirements and desired outcomes, it would be extremely beneficial for the user if the treatment protocol could instantaneously be tailored to the user's individual current physiological requirements.

It has been demonstrated that the efficiency of IHT sessions can be increased considerably if the control and adjustment of the oxygen concentration in hypoxic air, which is delivered to the user' respiratory system, is made to be directly dependent upon the individual user's physiological response. Thus, the present invention is directed to an apparatus for and method of IHT delivery to achieve this result wherein at least one of each individual user's physiological parameters, for example, his/her SpO₂, is used to determine the oxygen concentration in the hypoxic air delivered to that particular user. This automated biofeedback allows the oxygen concentration in the hypoxic air to be continuously adjusted according to a substantially constant pre-set SpO₂ target value. Thus, a SpO₂-driven exposure of a user to variable oxygen concentration allows the system to deliver an individually tailored exposure to each user regardless of his/her current physiological state, age, sex and other conditions.

It is an object of the present invention therefore to provide an improved apparatus for and method of delivery of IHT to a mammal, wherein the method overcomes or alleviates the disadvantages of the prior art.

### Summary of the Invention

According to the present invention, there may be provided an apparatus for the delivery of hypoxic air to a user comprising a biofeedback means wherein at least one physiologically measurable parameter of the user is substantially constantly measured by a monitoring means and the measured data transmitted to a control means, wherein the control means comprises:
i) means for comparing the measured data of the at least one physiological parameter with a pre-set target value for the parameter; and
ii) adjustment means to vary the oxygen concentration in the hypoxic air delivered to the user in response to the transmitted data.

Preferably, the physiologically measurable parameter is selected from the group of arterial oxygen saturation (SaO₂), heart rate (HR), blood pressure, ECG, cardiac output, exhaled CO₂, exhaled nitric oxide, breathing frequency, ventilation or any combination of any one or more of these parameters.

More preferably, the control means further comprises data input means for entering the pre-set target value for the user; data recording means for recording the target value for the user measured by the monitoring means; data transmitting means for transmitting the recorded measured data to a data comparing means, wherein the target value of step i) is compared with the measured value of step ii); and output means for transmitting a control signal to the adjustment means in response to the compared data of step iii), wherein if the measured value is below the target value, the adjustment means increases the oxygen concentration in the hypoxic air supplied to the user or if the measured value is above the target value, the adjustment means decreases the oxygen concentration in the hypoxic air supplied to the user.

The physiologically measured parameter of choice is the pulse oximeter measured arterial oxygen saturation (SpO₂) but may be any parameter that is suitable for the present invention.

The adjustment means preferably comprises at least one variable orifice mixer, where the greater the size of the orifice the lower the oxygen concentration in the produced hypoxic air delivered to the user. The adjustment means is most preferably electronically operable.

The apparatus of the invention preferably further comprises a hypoxic air generator for producing hypoxic air which is connected to the control means, where the generator responds to an output instruction received from the control means. Preferably, the hypoxic air generator comprises at least one of an air separation device or a pressure swing adsorption device to vary the oxygen concentration of the hypoxic air produced thereby.

If the device used is the air separation device, this may comprise a semi-permeable membrane supported therein and a pump for pumping intake air across the membrane, wherein the membrane separates the mixture into an oxygen-reduced gas mixture which is supplied to the user and an oxygen-rich gas mixture which is vented externally of the user or which is recyclable through the air separation device. Alternatively, the air separation device comprises a pressure swing adsorption device and a pump for pumping intake air across the pressure swing adsorption device. The pressure adsorption device preferably comprises molecular sieve material, more preferably a zeolite, which absorbs nitrogen from the mixture being compressed by the pump, leaving the oxygen-rich gas mixture, which is vented externally to the user or which may be transmitted to the user as required. More preferably, when the adsorption device is depressurised, a nitrogen concentrate gas is transmitted as the oxygen-reduced gas mixture to the user.

The control means of the invention further preferably comprises a flow control valve which is located inside the control means, which valve controls the flow rate of hypoxic air and thereby the oxygen concentration delivered to the user. The control valve is more preferably variable with respect to the concentration of oxygen delivered.

The intake air is preferably passed through at least one other piece of equipment selected from the group of an air pump, an air dryer and a moisture trap or any combination of one or more of said equipment before being fed through the semi-permeable membrane or the pressure swing adsorption device to produce hypoxic air.

In a preferred embodiment of the invention, the hypoxic air emitted from the membrane is fed into a 3/2 port switch, whereby the hypoxic air delivered to the user can be selected from hypoxic air or hyperoxic air.

The apparatus of the invention further comprising a respiratory circuit comprising a reservoir or breathing bag connected to the source of hypoxic air; a blow-off valve to prevent the bag from over-inflating; a demand valve for connecting the user to the external environment thereof whereby the respiratory circuit is interrupted; and a delivery means to deliver the hypoxic air to the user.

In an alternative use of the apparatus of the present invention, the first variable orifice mixer can be a fixed orifice mixer in order to keep the concentration of the oxygen in the hypoxic air emitted from the membrane substantially constant.

In yet a further preferred embodiment, there is a feeder pump connected to the source of hypoxic air to create a negative pressure towards the supply or storage of hypoxic air as well as to ambient air from the external environment, such that the hypoxic air and the ambient air form a mixture suitable for use in the invention as required. More preferably, the flow rate of the air mixture emitted from the feeder pump is greater than the flow rate of the hypoxic air emitted from the hypoxic air generator, if the hypoxic air is supplied under some pressure. There is preferably a second variable orifice valve located between the 3/2 port switch and the feeder pump to facilitate the mixing of the ambient air with the hypoxic air, thereby providing further means to control the oxygen concentration of the hypoxic air delivered to the user.

The monitoring means is more preferably selected from the group of a pulse oximeter, a blood pressure monitor, an electrocardiograph, a spirometer, an oxygen analyser, a capnometer, a nitric oxide meter and the like or any combination or series of any one or more of said means.

Generally, the oxygen concentration in the hypoxic air is varied in the range of between 15% and 8% by volume.

The scope of the present invention also extends to a method for the delivery of Intermittent Hypoxic Training (IHT), comprising the delivery of hypoxic air to a user utilising the apparatus of the invention, wherein at least one physiologically measurable parameter of the user is substantially constantly monitored and is used as a biofeedback means to vary the oxygen concentration in the hypoxic air delivered to the user as required, while keeping the chosen parameter substantially constant. The oxygen concentration in the hypoxic air is preferably varied between 15 % and 8% by volume.

The IHT is conducted over a sufficient number of training sessions to substantially improve the fitness of the user thereof and is individually designed or tailored for each individual's personal requirements.

### Brief Description of the Drawings

Figure 1 shows a schematic representation of IHT devices of the prior art;
Figure 2 shows results of tests of two different subjects receiving the same level of hypoxic air composed of 12 % of oxygen by volume for the same time period, where the top curve represents SpO₂ and the bottom curve represents HR;
Figure 3 shows the results of tests conducted on subject "Bart" receiving hypoxic air composed of 11 % of oxygen by volume, where the top curve represents SpO₂ and the bottom curve represents HR;
Figure 4 shows results of tests conducted on subject "Matt" receiving hypoxic air composed of oxygen by volume, where the top curve represents SpO₂ and the bottom curve represents HR;
Figure 5 is a schematic diagram of the device for the delivery of hypoxic air utilised in the method of the present invention;
Figure 6 is a schematic diagram of the apparatus of the invention showing the Variable Orifice Mixer;
Figure 7 is a schematic diagram of the apparatus of the invention showing the incorporation of the semipermeable air separator mechanism;
Figure 8 is a schematic diagram showing the PSA air separator mechanism of the invention;
Figure 9 is a schematic diagram showing pre-treatment of air fed into the Hypoxic Air Generator;
Figure 10 is a schematic diagram showing the Fixed Orifice Control Valve.

### Description of the Preferred Embodiment

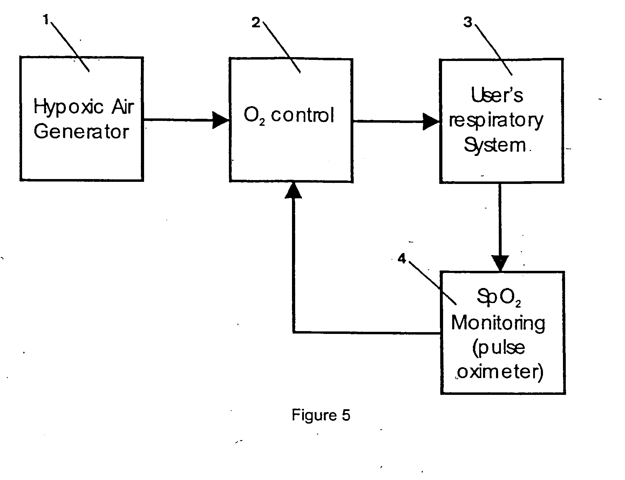

The invention will now be described, further explained and illustrated with reference to the following non-limiting example. Typically, the apparatus used in the treatment program of the present invention is described with particular reference to Figures 5 to 8 below as follows:

A schematic diagram of a preferred apparatus utilised in the method of the present invention is provided in Figure 5, which shows a Hypoxic Air Generator 1, which produces hypoxic air, the oxygen composition of which is controlled by a control mechanism, referred to as O₂ Control unit 2. The hypoxic air produced is then delivered to a User's Respiratory System 3. An arterial oxygen saturation (SpO₂) monitoring device 4 (pulse oximeter) monitors the user's individual response to the oxygen in the hypoxic gas mixture which the user has inspired (FiO₂) and if the user's SpO₂ is below or above the pre-determined and pre-set target SpO₂ value, a control signal is sent to the O₂ Control unit 2 by means of an O₂ Control device 5 that increases or decreases the oxygen content in the hypoxic air (FiO₂) delivered to the User's Respiratory System 3.

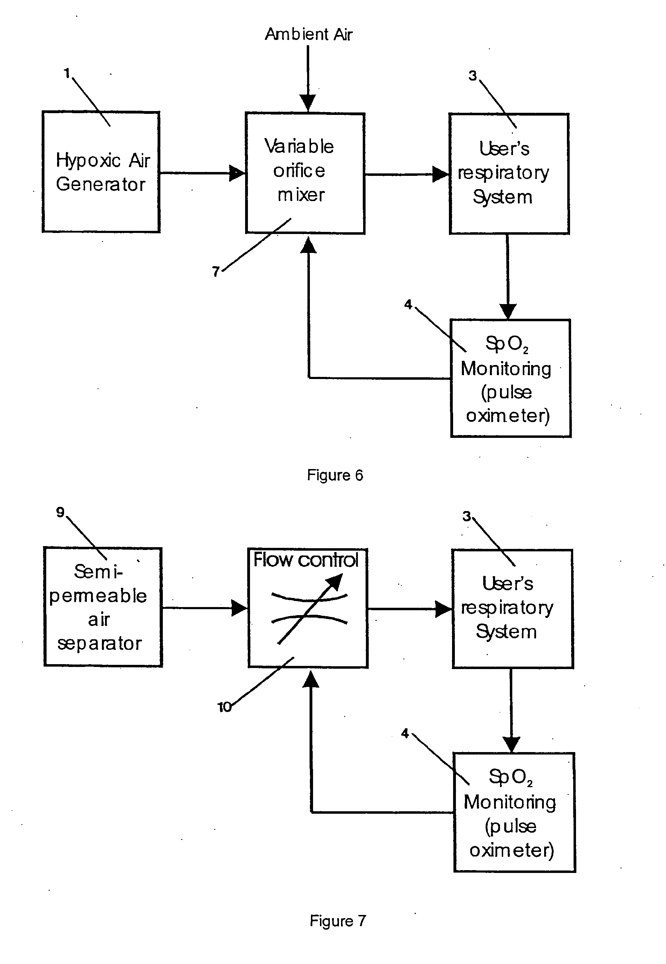

In one preferred embodiment of the present invention as illustrated in Figure 6, the Hypoxic Air Generator 1 comprises an air separation device 6 to control the amounts of oxygen and nitrogen constituting a pre-mixed gas mixture, which serves as the source of hypoxic air. In this way, the oxygen concentration can be fixed or varied as required. This hypoxic air is then delivered to the User's Respiratory System 3 via a Variable Orifice Mixer 7 that can further alter the composition of the hypoxic air by mixing the air produced by the Hypoxic Air Generator 1 with ambient air in order to generate a hypoxic air mixture having a higher oxygen concentration, which can then be delivered to the User's Respiratory System 3. The SpO₂ monitoring device 4 analyses the current value of the user's SpO₂. The SpO₂ monitoring device 4 further comprises means to enter and record the pre-determined and pre-set target value. If the user's current SpO₂ differs from the pre-set target value, a control signal is sent to the O₂ Control device 5, which, in a more preferred embodiment is a Variable Orifice Mixer 7 and most preferably, a Venturi Mixer 8, in order to adjust the oxygen concentration in the hypoxic air delivered to the user. The oxygen concentration in the gas mixture produced by the Hypoxic Air Generator 1 is adjusted so that if the SpO₂ value is higher than the target value, the oxygen concentration in the hypoxic air is decreased and if the SpO₂ is lower than the pre-set target value, then the oxygen concentration is increased.

The air separation device 6 for adjusting the oxygen concentration in the hypoxic air more preferably comprises a semipermeable membrane system 9 as shown in Figure 7. In this form of the device, the O₂ Control unit 2 further preferably comprises a Flow Control Valve 10 that is able to adjust the flow of hypoxic air produced by the semipermeable membrane system 9, whereby the oxygen concentration in the hypoxic air is directly proportional to the flow of the delivered hypoxic air. In response to a control signal delivered by the SpO₂ monitoring device 4, the oxygen concentration in the hypoxic air, which is delivered to the user, can be varied by varying one or more of the orifices of the Flow Control Valve 10.

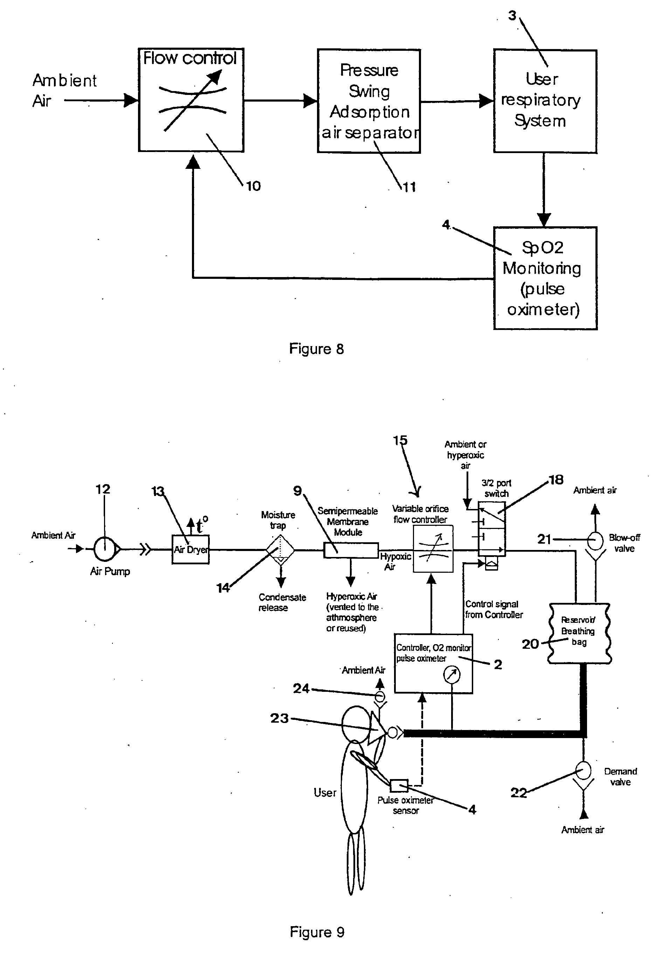

Yet another preferred embodiment of the apparatus of the invention is illustrated in Figure 8. In this embodiment, the Hypoxic Air Generator 1 utilises a Pressure Swing Adsorption (PSA) mechanism 11 to produce the hypoxic air. In this form of the apparatus, the SpO₂ monitoring device 4 sends a control signal to the Flow Control Valve 10 attached to the inlet of this device, which allows for adjustment of the oxygen concentration in the hypoxic air generated by the PSA mechanism 11.

Preferably, the air that is fed into the Hypoxic Air Generator 1 is compressed by an air pump 12, dried by an air drier 13 and dehumidified by a moisture trap 14 and then pumped through the semipermeable membrane system 9 as shown in Figure 9. The latter is designed so as to allow the compressed air to be divided into two streams namely, hyperoxic or hypoxic air. The hyperoxic air is either vented to the atmosphere or it can be used during the phase of IHT when the user receives ambient air in the form of hyperoxic air.

In this embodiment, the composition of the hypoxic air is controlled by a Variable Orifice Flow Controller 15, or alternatively, a proportional valve, which is preferably controlled by an electronic controller 17, which is connected to the O₂ Control unit 2. Preferably, the Variable Orifice Flow Controller 15 is motorised for greater efficiency and ease of use. The larger the orifice of the Variable Orifice Flow Controller 15, the lower the oxygen concentration in the produced hypoxic air.

In this way, the hypoxic air produced via a normally open 3/2 port switch 18 may be delivered to the User's Respiratory System 3. The apparatus of the invention further preferably comprises a User's Respiratory Circuit 19 having the following elements: a reservoir/breathing bag 20 connected to the source of hypoxic air; a Blow-off Valve 21 that prevents the breathing bag 20 from over-inflating; a Demand Valve 22 that connects the User or the User's Respiratory System 3 to the ambient air, in the case of a mismatch between the delivery of and demand for the hypoxic air or if the system is malfunctioning; means to deliver hypoxic air to the User's Respiratory System 3, preferably comprising an oxygen mask 23 with a Non-rebreathing Valve 24 connected to the mask 23; and supplies of both hypoxic air and ambient air, respectively.

The 3/2 port switch 18 is preferably incorporated for the purposes of switching the User's Respiratory System 3 from the delivery of hypoxic air to the delivery of ambient or a hyperoxic gas mixture.

The User's Respiratory System 3 is also connected to the O₂ Control unit 2 that monitors both the oxygen concentration in the hypoxic air delivered to the user as well as the user's physiological parameters, such as the SpO₂, HR, blood pressure, etc as described above. The Control device 5 sends a control signal to the Variable Orifice Flow Controller 15 that controls the oxygen concentration in the produced hypoxic air.

If the pre-set target value of SpO₂ is below the recommended value pre-determined for the user, then the O₂ Control unit 2 sends a signal to open the Variable Orifice Flow Controller 15 to increase the oxygen content in the hypoxic air delivered to the User's Respiratory System 3. Alternatively, if the SpO₂ value is above the desired value, the Variable Orifice Flow Controller 15 can be closed a fraction in order to deliver a lower oxygen concentration in the hypoxic air.

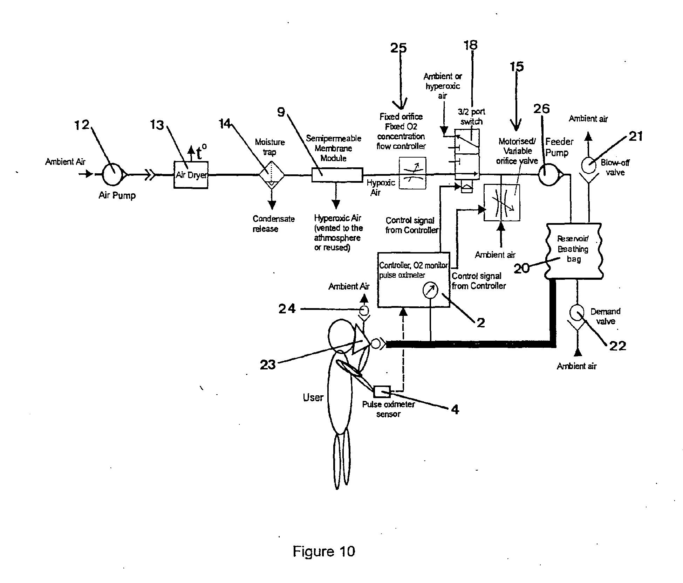

Yet another preferred embodiment of the apparatus of the present invention is illustrated in Figure 10.

In this form of the apparatus, there is a Fixed Orifice Control Valve 25 to enable the oxygen concentration in the hypoxic air produced to be maintained at a substantially constant level, which level is determined by the accuracy of the source of hypoxic air.

Preferably, the apparatus is additionally equipped with a Feeder Pump 26 to further assist with the control of the air composition and the breathing process of the user.

The oxygen composition in this apparatus is controlled by a Variable Orifice Flow Controller 15, or a proportional valve as an alternative controller, which may be motorised as before, but in this embodiment the controller 15 is connected between the vacuum port of the feeder pump 26 and the source of hypoxic air. The hypoxic air enters the feeder pump 26 through the vacuum port.

By varying the cross-sectional diameter of the controller 15 or in the alternative, a Multiple Orifice Valve 28, the O₂ Control unit 2 is able to alter the proportion in which the supplied hypoxic air is mixed with the ambient air and ultimately, therefore, the oxygen composition of the hypoxic air produced by the system.

The scope of the present invention also extends to a method of administering IHT to a mammalian user, which employs or incorporates the apparatus of the present invention in its IHT training regimen.

### Results after employing the method and apparatus of the invention

The method utilising the apparatus of the present invention was tested on Subject "Rosy". Her IHT training schedule as prescribed by her IHT practitioner is provided in Table 1, which records the number of the training session and the target SpO₂ set for that session.

**Table 1. Rosy's IHT Training Schedule**

| Session | Target SpO₂ (+/- 2% |
|---|---|
| 1 | 88 |
| 2 | 88 |
| 3 | 86 |
| 4 | 86 |
| 5 | 83 |
| 6 | 83 |
| 7 onwards | 80 |

### Results

Before starting on and on the completion of the program, various physiological parameters for "Rosy" were measured. These results are shown in Table 2 below.

**Table 2. Results of testing the IHT method of the invention on subject "Rosy"**

| **Parameter** | **Initial** | **Final** |
|---|---|---|
| Heart Rate (beats/min) | 68 | 62 |
| Blood Pressure (mm Hg) | 145/90 | 130/80 |
| Exercise till Exhaustion Time | 50W at 150 (beats/min) for 12 minutes | doubled |

Similar results on other users in Rosy's age group have previously only been seen after two or three courses of IHT, each course comprising 15 daily training sessions, when using the conventional protocol. These results therefore illustrate that the method of the present invention demonstrates higher efficacy, faster beneficial results and therefore a reduced number of courses and lower overall cost for the user.

Those skilled in the art will appreciate that while the invention described herein for simplicity's sake has referred to a human user, it has a general applicability to mammals in general. As a consequence, in another form of the invention, the method of treatment can be applied to an animal. It is to be understood that the invention includes all such variations and modifications. The invention also includes all the steps, features, concentrations, time periods, variations in hypoxic air generators, including air separation devices, semipermeable membranes, pressure adsorption devices, mixers, control valves, breathing regimens, respiratory circuits, breathing masks etc. referred to or indicated in the specification individually or collectively and any and all combinations of any two or more of said steps, devices or features.

Where the terms "comprise", "comprises", "comprised" or "comprising" are used in this specification, they are to be interpreted as specifying the presence of the stated features, integers, steps or components referred to, but nor to preclude the presence or addition of one or more other feature, integer, step, component or group thereof.

The claims defining the invention are as follows:

## Claims

1. An apparatus for the delivery of hypoxic air to a user comprising a biofeedback means wherein at least one physiologically measurable parameter of the user is substantially constantly measured by a monitoring means and the measured data transmitted to a control means, wherein the control means comprises:
i) means for comparing the measured data of the at least one physiological parameter with a pre-set target value for the parameter; and
ii) adjustment means to vary the oxygen concentration in the hypoxic air delivered to the user in response to the transmitted data.

2. An apparatus according to Claim 1, wherein the physiologically measurable parameter is selected from the group of arterial oxygen saturation (SaO₂), heart rate (HR), blood pressure, ECG, cardiac output, exhaled CO₂, exhaled nitric oxide, breathing frequency, ventilation or any combination of any one or more of these parameters.

3. An apparatus according to Claim 1 or Claim 2, wherein the control means further comprises:
i) data input means for entering the pre-set target value for the user;
ii) data recording means for recording the target value for the user measured by the monitoring means;
iii) data transmitting means for transmitting the recorded measured data to a data comparing means, wherein the target value of step i) is compared with the measured value of step ii); and
iii) output means for transmitting a control signal to the adjustment means in response to the compared data of step iii),
wherein if the measured value is below the target value, the adjustment means increases the oxygen concentration in the hypoxic air supplied to the user or if the measured value is above the target value, the adjustment means decreases the oxygen concentration in the hypoxic air supplied to the user.

4. An apparatus according to any one of Claims 1 to 3, wherein the physiological parameter is the arterial oxygen saturation (SpO₂).

5. An apparatus according to any one of Claims 1 to 4, wherein the adjustment means comprises at least one variable orifice mixer, wherein the greater the size of the orifice the lower the oxygen concentration in the produced hypoxic air delivered to the user.

6. An apparatus according to any one of Claims 1 to 5, further comprising a hypoxic air generator for producing hypoxic air connected to the control means, wherein the generator responds to an output instruction received from the control means.

7. An apparatus according to Claim 6, wherein the hypoxic air generator which comprises at least one of an air separation device or a pressure swing adsorption device to vary the oxygen concentration of the hypoxic air produced thereby.

8. An apparatus according to Claim 7, wherein the air separation device comprises a semi-permeable membrane supported therein and a pump for pumping intake air across the membrane, wherein the membrane separates the mixture into an oxygen-reduced gas mixture which is supplied to the user and an oxygen-rich gas mixture which is vented externally of the user or which is recyclable through the air separation device.

9. An apparatus according to Claim 7, wherein the air separation device comprises a pressure swing adsorption device and a pump for pumping intake air across the pressure swing adsorption device.

10. An apparatus according to Claim 8, wherein the control means further comprises a flow control valve located inside the control means, which valve controls the flow rate of hypoxic air and thereby the oxygen concentration delivered to the user.

11. An apparatus according to Claim 10, whereby the control valve is variable with respect to the concentration of oxygen delivered.

12. An apparatus according to Claim 9, wherein the pressure adsorption device comprises molecular sieve material, whereby nitrogen is adsorbed from the mixture being compressed by the pump, leaving the oxygen-rich gas mixture which is vented externally to the user or transmitted to the user as required.

13. An apparatus according to Claim 12, wherein when the adsorption device is depressurised, a nitrogen concentrate gas is transmitted as the oxygen-reduced gas mixture to the user.

14. An apparatus according to any one of Claims 8 to 13, wherein the intake air is passed through at least one other piece of equipment selected from the group of an air pump, an air dryer and a moisture trap or any combination of one or more of said equipment before being fed through the semi-permeable membrane or the pressure swing adsorption device to produce hypoxic air.

15. An apparatus according to Claim 14, wherein the hypoxic air emitted from the membrane is fed into a 3/2 port switch, whereby the hypoxic air delivered to the user is selected from hypoxic air or hyperoxic air.

16. An apparatus according to any one of claims 1 to 15, further comprising a respiratory circuit comprising:
i) a reservoir or breathing bag connected to the source of hypoxic air;
ii) a blow-off valve to prevent the bag from over-inflating
iii) a demand valve for connecting the user to the external environment thereof whereby the respiratory circuit is interrupted; and
iv) a delivery means to deliver the hypoxic air to the user.

17. An apparatus according to any one of Claims 5 to 16, wherein the first variable orifice mixer is a fixed orifice mixer to allow the concentration of the oxygen in the hypoxic air emitted from the membrane to be kept substantially constant.

18. An apparatus according to Claim 17, further comprising a feeder pump connected to the source of hypoxic air.

19. An apparatus according to Claim 17 or Claim 18, further comprising a second variable orifice valve located between the 3/2 port switch and the feeder pump to allow mixing of an amount of ambient air with the hypoxic air, thereby altering the oxygen concentration of the hypoxic air delivered to the user.

20. An apparatus according to any one of Claims 1 to 19, wherein the monitoring means is selected from the group of a pulse oximeter, a blood pressure monitor, an electrocardiograph, a spirometer, an oxygen analyser, a capnometer, a nitric oxide meter or the like or any combination or series of any one or more of said means.

21. An apparatus according to any one of Claims 1 to 20, wherein the adjustment means is electronically operable.

22. An apparatus according to any one of Claims 1 to 21, wherein the oxygen concentration in the hypoxic air is varied in the range of between 15% and 8% by volume.

23. A method for the delivery of Intermittent Hypoxic Training (IHT), comprising the delivery of hypoxic air to a user utilising the apparatus of any one of Claims 1 to 22, wherein at least one physiologically measurable parameter of the user is substantially constantly monitored and is used as a biofeedback means to vary if required the amount of oxygen concentration in the hypoxic air delivered thereto, whereby the at least one chosen parameter is kept substantially constant.

24. A method according to Claim 23, wherein the oxygen concentration in the hypoxic air is varied between 15% and 8% by volume.

25. A method according to Claim 23 or Claim 24, wherein the Training is conducted over a sufficient number of training sessions to substantially improve the fitness of the user thereof

## Amended claims

### Amended claims in accordance with Rule 86(2) EPC.

**1.** An apparatus for the delivery of hypoxic air to a user comprising a biofeedback means, wherein the biofeedback means includes a first monitoring means and a control means, wherein the monitoring means substantially constantly measures the user's arterial oxygen saturation (SaO₂) during a training session and transmits the measured SaO₂ value to the control means, which control means conducts a comparison between the measured SaO₂ value and a pre-set target SaO₂ value and wherein the control means by an adjustment means varies the oxygen concentration in the hypoxic air being delivered to the user so that, if the SaO₂ value is higher than the target value, the oxygen concentration in the hypoxic air is decreased, and if the SaO₂ value is lower than the pre-set target value, then the oxygen concentration is increased.

**2.** An apparatus according to Claim 1, wherein the control means further comprises:
i) data input means for entering the pre-set target SaO₂ value for the user;
ii) data recording means for recording the SaO₂ value for the user measured by the monitoring means;
iii) data transmitting means for transmitting the recorded measured value to a data comparing means, wherein the measured SaO₂ value is compared with the pre-set target SaO₂ value; and
iv) output means for transmitting a control signal to the adjustment means in response to the compared data of step iii),
wherein if the measured SaO₂ value is below the pre-set target SaO₂ value, the adjustment means increases the oxygen concentration in the hypoxic air supplied to the user or if the measured SaO₂ value is above the pre-set target SaO₂ value, the adjustment means decreases the oxygen concentration in the hypoxic air supplied to the user.

**3.** An apparatus according to Claim 1 or Claim 2, wherein the adjustment means comprises at least one variable orifice mixer, wherein the greater the size of the orifice of the mixer the lower the oxygen concentration in the produced hypoxic air delivered to the user.

**4.** An apparatus according to any one of claims 1, 2 or 3, further comprising a hypoxic air generator connectable to the control means for producing hypoxic air, wherein the generator responds to an output instruction received from the control means.

**5.** An apparatus according to Claim 4, wherein the hypoxic air generator comprises at least one of an air separation device or a pressure swing adsorption device to vary the oxygen concentration of the hypoxic air produced thereby.

**6.** An apparatus according to Claim 5, wherein the air separation device comprises a semi-permeable membrane supported therein and a pump for pumping intake air across the membrane, wherein the membrane separates the intake air into an oxygen-reduced gas mixture which is supplied to the user and an oxygen-rich gas mixture which is vented externally of the user or which is recyclable back through the air separation device.

**7.** An apparatus according to Claim 5, wherein the air separation device comprises a pressure swing adsorption device and a pump for pumping the intake air across the pressure swing adsorption device.

**8.** An apparatus according to Claim 7, whereby the control means further comprises a flow control valve, which valve controls the flow rate of hypoxic air.

**9.** An apparatus according to Claim 7 or Claim 8, wherein the pressure adsorption device comprises molecular sieve material, whereby nitrogen is adsorbed from the intake air being compressed by the pump, leaving an oxygen-rich gas mixture which is vented externally to the user or which may be transmitted to the user as required.

**10.** An apparatus according to Claim 9, wherein when the adsorption device is depressurised, a nitrogen concentrate gas is transmitted to the user as the oxygen-reduced gas mixture.

**11.** An apparatus according to any one of Claims 6 to 10, wherein the intake air is passed through at least one other piece of equipment selected from the group of an air pump, an air dryer and a moisture trap or any combination of one or more of said equipment before being fed through the semi-permeable membrane or the pressure swing adsorption device to produce hypoxic air for delivery to the user and hyperoxic air for venting to the atmosphere or for re-use.

**12.** An apparatus according to Claim 11, wherein the hypoxic and hyperoxic air emitted from the membrane is fed into a 3/2 port switch, whereby the air delivered to the user is selected from hypoxic air or hyperoxic air.

**13.** An apparatus according to any one of claims 1 to 12, further comprising a respiratory circuit comprising:
i) a reservoir or breathing bag connected to the source of hypoxic air;
ii) a blow-off valve to prevent the bag from over-inflating;
iii) a demand valve for connecting the user to the external environment thereof whereby the respiratory circuit is interrupted; and
iv) a delivery means to deliver the hypoxic air to the user.

**14.** An apparatus according to any one of Claims 6 to 13, wherein the first variable orifice mixer is a fixed orifice mixer to allow the concentration of the oxygen in the hypoxic air emitted from the membrane to be kept substantially constant.

**15.** An apparatus according to Claim 14, further comprising a feeder pump connected to the source of hypoxic air.

**16.** An apparatus according to Claim 14 or Claim 15, further comprising a second variable orifice valve located between the 3/2 port switch and the feeder pump to allow mixing of an amount of ambient air with the hypoxic air, thereby altering the oxygen concentration of the hypoxic air delivered to the user.

**17.** An apparatus according to any one of Claims 1 to 16, wherein the monitoring means is a pulse oximeter.

**18.** An apparatus according to any one of Claims 1 to 17, wherein the measured SaO₂ value is combined with measurement by a monitoring means of at least one physiologically measurable parameter selected from heart rate (HR), blood pressure, ECG, cardiac output, exhaled CO₂, exhaled nitric oxide, breathing frequency or ventilation.

**19.** An apparatus according to Claim 18, wherein the monitoring means is selected from a blood pressure monitor, an electrocardiograph, a spirometer, an oxygen analyser, a capnometer, a nitric oxide meter or the like or any combination or series of any one or more of said means.

**20.** An apparatus according to any one of Claims 1 to 19, wherein the adjustment means is electronically operable.

**21.** An apparatus according to any one of Claims 1 to 20, wherein the oxygen concentration in the hypoxic air is varied in the range of between 15 % and 8 % by volume.
